# EUROPEAN PATENT APPLICATION

(11) **EP 4 595 776 A1**
(43) Date of publication of application: **06.08.2025**
(21) Application number: 24155475.7
(22) Date of filing: 02.02.2024
(51) Int. Cl.: A23L 33/105, A23L 33/12, A23L 33/155, A23L 33/16, A23L 33/175, A61K 33/04, A61K 33/24, A61K 31/05, A61K 31/185, A61K 31/201, A23L 33/00

(54) **NUTRITIONAL COMPOSITION FOR CANCER PATIENTS AND ELDERLY SUBJECTS**

(71) Applicant: Pasqualetti, Giuseppe, 56123 Pisa (IT); Loupakis, Fotios, 01100 Viterbo (IT)
(72) Inventor: Pasqualetti, Giuseppe, 56123 Pisa (IT); Loupakis, Fotios, 01100 Viterbo (IT)
(74) Representative: Pace Napoleone, Maria

(57) **Abstract**

The invention relates to the nutritional supplementation of a specific category of cancer patients or elderly subjects as defined by low levels of serum fT3/fT4 ratio.

The supplement is made of specific dosages of vitamin A, resveratrol, oleic acid, taurine and selenium combined to synergistically act for increasing fT4 to fT3 hormones peripheral conversion operated by deiodinase 1 enzyme by stimulating its expression and to provide an optimal metabolic support to subjects at high risk of nutrition-related worsening of their clinical conditions.

## Description

### Field of the invention

The present invention relates to a supplement aimed at improving clinical conditions through re-establishing a more physiological and favourable metabolic status of 1) patients suffering from cancer or of 2) elderly subjects, at high risk of malnutrition, identified by detecting a low degree of conversion of free thyroxine (fT4) to free triiodothyronine (fT3).

### Background Art

In the realm of modern healthcare, nutritional inadequacy or deficiency up to relevant malnutrition remains silent yet important challenge, particularly among cancer patients and elderly subjects. Recent studies indicate that up to 85% of cancer patients may experience malnutrition at some stage of their illness. Studies around the world showed prevalence of malnutrition among elderly ranging from 13% to 54%. Malnutrition in these vulnerable groups is often a result of multiple factors, including decreased appetite, the physical and emotional toll of illness, and the side effects of treatments. The consequences of malnutrition are profound, ranging from weakened immune responses and muscle wasting to impaired recovery and increased mortality rates. This issue not only affects the quality of life for patients, but also poses significant challenges for healthcare systems worldwide. The deterioration of health due to malnutrition leads to longer hospital stays, increased healthcare costs, and a higher burden on caregivers. In light of these challenges, the concept of proactive measures - such as prehabilitation intervention - to prepare patients for the physical and psychological stresses of their illness and related treatment - emerges as a beacon of hope. Specific nutritional support and dietary advice, physical exercise, and psychological counselling should be tailored and implemented to bolster patients' strength and resilience. Cancer-associated malnutrition is a common condition and it can result from local effects of a tumour, the host response to the tumour and the anticancer therapies themselves. Moreover, although cancer patients often have reduced food intake (due to systemic effects of the disease, local tumour effects, psychological effects or adverse effects of treatment), alterations in nutrient metabolism and resting energy expenditure may also contribute to nutritional status. Several agents produced by the tumour directly, or systemically in response to the tumour, such as pro-inflammatory cytokines and hormones, have been implicated in the pathogenesis of malnutrition and cachexia. The consequences of malnutrition include impairment of immune functions, performance status, muscle function, and quality of life. In addition, responses to chemotherapy are decreased, chemotherapy-induced toxicity and complications are more frequent and severe, and survival times are shortened (Van Cutsem E, et al. Eur J Oncol Nurs. 2005).

Current guidelines for clinical nutrition in cancer from the European Society for Clinical Nutrition and Metabolism recommend "to ensure an adequate nutritional intake and to maintain physical activity" during anticancer drug treatment (Muscaritoli M, et al. Clin Nutr. 2021). There are no specific laboratory abnormalities for the selection of patients nor specific nutritional approaches recommended. The same considerations apply to the treatment of muscle loss in elderly subjects for which there are no specific metabolic biomarkers nor metabolic pathway targets for identifying patients at risk of malnutrition.

To date, there are no therapeutic strategies or supplements conceived for acting on specific central mechanisms responsible for regulating a patient's metabolism in its entirety as a complex organism.

Cancer cachexia is quite common in patients with advanced cancer as well as one of its major phenotypic signs, i.e. decreased skeletal muscle mass, a condition known as sarcopenia. The guidelines on cancer cachexia from the European Society of Medical Oncology recommend early intervention with nutritional support in patients with signs of cachexia (Arends J, et al. ESMO Open. 2021). Similarly, intervention for sarcopenia in elderly includes exercise and nutritional therapy (Ali et al. Gerontology. 2014).

Recent evidence correlates sarcopenia to levels of total T3 below the normal value (Chen J, et al. Clin Interv Aging. 2023). Nonetheless, single measurements of fT3 or fT4 may not be as accurate prognosticators as their ratio. In fact, while T4 stands out as the predominant thyroid hormone in the bloodstream, the primary impact of thyroid hormones is attributed to T3. The majority of circulating T3 originates from the conversion of peripheral T4 facilitated by deiodinase. Therefore, the conversion from T4 to T3 plays a pivotal role in generating circulating T3. This process can be assessed through the fT3/fT4 ratio. In recent studies, the fT3/fT4 ratio emerged as a potentially more accurate and practical gauge of thyroid hormone metabolic variations, demonstrating a stronger correlation with prognosis compared to individual measurements of fT3 or fT4 in elderly patients (Pasqualetti G, et al. J Clin Endocrinol Metab. 2018). For further clarifying, fT3/fT4 ratio prognostic relevance is equally retained when the free moieties of thyroid hormones are within their normal range of values. fT3/fT4 ratio was also strongly correlated to the so-called Multi Prognostic Index (or MPI), an indirect measure of frailty. Given that cancer progression and neoplastic cachexia physiologically resemble what is observed in the frailty syndrome, which is a well-known and extensively described multifactorial clinical entity resulting in increased catabolism and muscle wasting, the initial observations in elderly patients paved the way for further studies in oncologic patients.

To date no therapeutic intervention nor specific nutritional approaches or specific supplements have been developed to sustain fT4 to fT3 conversion in cancer patients or elderly patients at risk of malnutrition. Taking into account: i) the central role of fT3/fT4 ratio on the prognosis of those subjects and ii) the negative effects of a reduced peripheral thyroid hormones conversion and the subsequent not-physiologic fT3 levels (even if in the normal range of normality) on metabolism and vital functions, the administration of core elements that support deiodinase activity, associated or not to other nutrients, is an innovative approach for the nutritional support or treatment of such patients.

### SUMMARY OF THE INVENTION

The inventors of the present invention focused on a specific aspect of thyroid function moving from initial observations conducted in elderly hospitalised patients and showing that fT3/fT4 ratio was a major determinant of prognosis (Pasqualetti G, et al. J Clin Endocrinol Metab2018).

Moving from the hypothesis that downregulation of fT3/fT4 ratio could be a crucial mechanism of adaptation to a stressful condition in various diseases, the inventors explored the prognostic relevance of the ratio in advanced cancer patients.

Strong clinical research has been conducted to definitively demonstrate the centrality and clinically significant effect of the balance between thyroid hormones and survival in advanced cancer patients. Initial findings have been published by the inventors in prestigious journals (Schirripa M, et al. Clin Colorectal Cancer. 2018; Pasqualetti G, et al. Eur J Cancer. 2020). Additional data on file were produced to replicate the evidence in other cancer settings, the fT3/fT4 ratio is a strong adverse prognosticator in advanced cervical cancer patients, advanced breast cancer patients, advanced lung cancer patients. New data on file produced by the inventors confirm for the first time in a cohort of 11 metastatic cancer patients a significant correlation of baseline CT-scan diagnosed condition of sarcopenia with lower fT3/fT4 ratio (N=6 sarcopenic subject vs n=5 non sarcopenic, median fT3/fT4 ratio of 0.200 (range 0.109-0.283) and 0.278 (range 0.250-0.301) respectively, Mann-Whitney U-test, p=0.027).

The present invention was made in order to address the issue of worse prognosis in patients with low fT3/fT4 ratio and its correlation with sarcopenia providing them with a specific nutritional supplement to be administered at the time of diagnosis in advanced stage cancer patients or when there is a high risk for malnutrition in elderly subjects.

It is therefore an object of the invention a nutritional supplement developed to act on 4 specific levels in order to sustain an adequate balance of peripheral thyroid hormones:
a. Support deiodinase 1 gene expression and enable deiodinase proteins synthesis.
b. Provide specific trace elements for deiodinase proteins synthesis.
c. Reduce the negative impact of inflammation on deiodinase expression.
d. Enhance immune system and improve muscle trophism.

The following components were selected in order to provide a synergistic and optimised effect at the above reported levels of action:
1. Vitamin A,
2. Resveratrol,
3. Monounsaturated fatty acids,
4. Taurine,
5. Selenium.

### DETAILED DESCRIPTION OF THE INVENTION

The present invention provides a novel nutritional supplement specifically conceived to guarantee tailored nutritional support to cancer patients and elderly subjects in specific metabolic need and at higher risk of earlier death as defined by low fT3/fT4 ratio. In the course of the invention it was identified that vitamin A, resveratrol, oleic acid, taurine, selenocysteine synergistically increase fT4 to fT3 hormones peripheral conversion operated by deiodinase 1 enzyme by stimulating its expression and providing an optimal metabolic support to subjects at high risk of nutrition-related worsening of their clinical conditions.

It is an object of the invention a composition or combination comprising or consisting of:
o vitamin A; and
o resveratrol or derivatives thereof; and
o a monounsaturated fatty acid or mixtures thereof;
o taurine or taurine-like substances; and
o selenium or a selenium-based compound, preferably being selected from a selenate salt and seleno-amino acid.

Preferably, the resveratrol is comprised in parts of a plant or in an extract of said parts of plant, wherein said plant is selected from Vitis vinifera, Polygonum cuspidatum, Blueberries (Vaccinium spp.), Mulberries (Morus spp.), Cranberries (Vaccinium macrocarpon), and Aronia (Photinia melanocarpa).

Preferably, the monounsaturated fatty acid is selected from oleic acid, palmitoleic acid and gadoleic acid or mixtures thereof; still preferably the seleno-aminoacid is selected from selenocysteine and selenomethionine, preferably the seleno-aminoacid is selenocysteine.

In a preferred embodiment, the composition or combination of the invention comprises:
- about 125 to 1000 mcg of vitamin A, preferably about 500 to 1000 mcg; and/or
- about 50 to 500 mg of resveratrol, preferably about 300 to 500 mg; and/or
- about 1.5 to 7 g of oleic acid, preferably about 3 to 7 mg; and/or
- about 100 to 1000 mg of taurine; and/or
- about 30 to 250 mcg, of selenocysteine, preferably about 100 to 250 mcg.

In a further preferred embodiment, the composition or combination of the invention comprises:
- about 750 mcg of vitamin A; and/or
- about 400 mg of resveratrol; and/or
- about 5 g of oleic acid; and/or
- about 500 mg of taurine; and/or
- about 200 mcg of selenocysteine.

It is a further object of the invention, the composition or combination as defined above further comprising one or more ingredients selected from: vitamin D, iron (Fe), zinc (Zn) and iodine (I).

Additionally, in a preferred embodiment the composition or combination also comprises one or more ingredients selected from: copper (Cu), manganese (Mn), fluorine (F), molybdenum (Mo), chromium (Cr), vitamin E, vitamin K, thiamine (vitamin B1), riboflavine (vitamin B2), niacine (vitamin B3), panthotenic acid, vitamin B6, folic acid, vitamin B12, biotin, vitamin C and choline.

It is a further object of the invention the composition or combination as above defined further comprising or in combination with a nutritional supplement (nutritional "backbone") comprising proteins, carbohydrate and fatty acids, preferably said nutritional supplement comprises proteins in concentration of about 10g/100 mL (ranging from 5 to 30g), fatty acids in concentration of about 5g/100 mL (ranging from 0 to 20g) and carbohydrates in concentration of about 20g/100 mL (ranging from 0 to 30g).

Preferably the composition of the invention comprises at least one excipient or diluent, preferably for oral administration, preferably in the form of a lozenge, a hard capsule, a soft gel capsule, a powder, a syrup, a cachet, a pill, a tablet, a pastille, preferably said excipient and/or diluent, is selected from the group consisting of: calcium phosphate, dicalcium phosphate, microcrystalline cellulose, silicon dioxide, sucrose, gum arabic, corn starch, medium chain triglycerides, tricalcium phosphate, cross-linked sodium carboxymethylcellulose, hydroxypropylmethylcellulose, polyethylene glycol, titanium dioxide, polyvinylpyrrolidone, talc, erythritol, xylitol, steviol and sucralose glycosides, magnesium salt of fatty acids, preferably said magnesium salt of fatty acids is magnesium stearate.

It is a further object of the invention a pharmaceutical composition, medical device or supplement or food product, preferably a food for special medical purposes, or product to drink, comprising the composition of the invention.

Preferably said composition or said pharmaceutical composition, medical device or supplement or food product, preferably a food for special medical purposes, or product to drink is for use in the nutritional support or treatment of patients affected by cancer and at high risk of malnutrition as defined by level of fT3/fT4 ratio below the mean reference values for said subjects.

Preferably said composition or said pharmaceutical composition, medical device or supplement or food product, preferably a food for special medical purposes, or product to drink is for use in the nutritional support or treatment of elderly subjects at high risk of malnutrition as defined by level of fT3/fT4 ratio below the mean reference values for said subjects.

Preferably, the composition of the invention or the pharmaceutical composition, medical device or supplement or food product, preferably a food for special medical purposes, or product to drink is characterised in that it supports deiodinase 1 gene expression and enable deiodinase proteins synthesis and/or provides specific trace elements for deiodinase proteins synthesis and/or reduces the negative impact of inflammation on deiodinase expression and/or enhance immune system and improve muscle trophism and/or sustains fT4 to fT3 peripheral conversion.

Preferably the composition of the invention or the pharmaceutical composition, medical device or supplement or food product, preferably a food for special medical purposes, or product to drink is for use in the treatment of cancer or as a food supplement having beneficial effects in cancer patients, wherein said cancer includes breast cancer, colorectal cancer, esophageal cancer, liver cancer, pancreatic cancer, stomach (gastric) cancer, endocrine system cancer including thyroid cancer, adrenal cancer and pituitary cancer, female reproductive system cancer, including ovarian cancer, cervical cancer and uterine (endometrial) cancer, head and neck cancer, oral cancer, throat cancer, lung cancer, mesothelioma, male reproductive system cancer, including prostate cancer and testicular cancer, musculoskeletal system cancer, including bone cancer and soft tissue sarcoma, skin cancer, including melanoma and non-melanoma skin cancer such as basal cell carcinoma and squamous cell carcinoma, urinary system cancer including bladder cancer and kidney cancer, central nervous system cancer including brain tumours and spinal cord tumours, eye cancer (retinoblastoma), immune system cancer including Hodgkin lymphoma and non-Hodgkin lymphoma.

It is a further object of the invention the non-therapeutic use of the composition as above defined in the nutraceutical sector or as base ingredient in the preparation of supplements and/or devices and/or food products and/or foods for special medical purposes.

It is a further object of the invention the composition or the pharmaceutical composition, medical device or supplement or food product, preferably a food for special medical purposes, or product to drink for use in combination with a further therapeutic agent or therapeutic intervention or with nutritional supplement. The following paragraphs define various terms and expressions used in the application to describe the invention.

*Vitamin A* - also known as retinol or retinoic acid, is a nutrient important to vision, growth, cell division, reproduction and immunity. Vitamin A also has antioxidant properties. Studies support the evidence that vitamin A could enhance the hepatic activation of T4 into T3 by increasing the activity of deiodinases (Type 1 and Type 2) and finally decreasing the serum level of T4 (Zimmermann MB, et al. J Clin Endocrinol Metab. 2004; Morley JE, et al Am J Physiol. 1980; Saleh et al. EJ Nutrition 2022).

Vitamin A in the compositions provided herein can include, without limitation, one or more of beta-carotene, retinol, retinaldehyde, retinoic acid, and salts thereof. For example, vitamin A can include one or more of beta-carotene, retinyl palmitate, or retinyl acetate. In some embodiments, the composition includes vitamin A as retinyl palmitate. In some embodiments, the composition includes vitamin A as beta-carotene. When vitamin A is present in the compositions herein, the compositions can include vitamin A in an amount of at least about 5% of the U.S. Recommended Daily Intake (USRDI) of vitamin A. The U.S. Recommended Daily Intake (USRDI) for vitamins and minerals are defined and set forth in the Recommended Daily Dietary Allowance-Food and Nutrition Board, National Academy of Sciences-National Research Council. In some embodiments, vitamin A is present in the compositions provided herein in an amount of about 10% to about 200% of the USRDI of vitamin A. For example, vitamin A can be present in the compositions provided herein in an amount of about 5% to about 150%, about 10% to about 150%, or about 20% to 125%, or about 30% to about 100%, or about 30% to about 75% or about 50% of the USRDI of vitamin A. In some embodiments, when vitamin A is present in a composition provided herein, the composition can include vitamin A in an amount of at least about 25 mcg RAE, wherein the term "mcg RAE" refers to micrograms of retinol activity equivalents. For example, vitamin A can be present in the compositions provided herein in an amount of about 50 mcg RAE to about 1500 mcg RAE, about 100 mcg RAE to about 1000 mcg RAE, about 125 mcg RAE to about 850 mcg, about 250 mcg RAE to about 800 mcg, RAE about 500 mcg RAE to about 750 mcg RAE. In some cases, vitamin A is present in the composition in an amount of about 125 mcg RAE, about 250 mcg RAE, about 750 mcg RAE.

*Resveratrol* - Resveratrol, with the chemical name 3,5,4'-trihydroxy-trans-stilbene, is a plant compound belonging to the stilbenes and is found in high concentrations in the skin of red grapes, berries, nuts and other plant products. According to the invention, resveratrol can be used as such or comprised in parts of a plant or in an extract of said parts of plant, wherein preferably said plant is selected from Vitis vinifera, Polygonum cuspidatum, Blueberries (Vaccinium spp.), Mulberries (Morus spp.), Cranberries (Vaccinium macrocarpon), and Aronia (Photinia melanocarpa).

As used herein, the term "resveratrol" describes 3,5,4'-trihydroxy-trans-stilbene as well as isomeric compounds and derivatives. The term "derivatives" as used herein embraces the sources of natural occurring hydroxylated stilbenes or stilbenoids including the methyl ethers and glycosides thereof. Preferred resveratrol derivatives are resveratrol, oxy-resveratrol also known as piceatannol, the methylated stilbenoids selected from the group consisting of 4-methoxyresveratrol, gnetucleistol D (2-methoxyoxyresveratrol), gnetucleistol E (3-methoxy-isorhapontigenin), isorhapontigenin (3,4',5-trihydroxy-3'-methoxystilbene), pinostilbene (3-methoxyresveratrol), pterostilbene (3',5'-dimethoxyresveratrol), rhapontigenin (piceatannol 4'-methyl ether), combretastatin A-I and combretastatin A-4, and the glycosylated stilbenoids selected from the group consisting of piceid (trans-resveratrol-3-O-glucoside), trans-resveratrol-3-O-glucuronide, resveratroloside (trans- resveratrol-4'-0-beta-D-glucopyranoside), rhapontigenin 3-O-rutinoside, 4'-methoxy-(E)-resveratrol 3- O-rutinoside and rhaponticin (rhapontigenin glucoside). It is well known that resveratrol is almost insoluble in water and has stability issues. It has been suggested that the solubility and stability of resveratrol in water could be increased with the aid of a dendrimer nanotechnology or liposomal resveratrol preparations (Petek T, et al. Molecules 2017). In addition, the solubility of resveratrol in water may be enhanced by associating it with carboxymethyl beta-glucan as suggested by the European Patent EP 2674 155. In an exemplary embodiment, 0.001 to 20% by weight of a resveratrol derivative are present in a pharmaceutical unit dose according to the invention. In an exemplary embodiment, 0.05 to 10% by weight of a resveratrol derivative are present in a unit dose according to the invention. In an exemplary embodiment, 0.1 to 8% by weight of a resveratrol derivative are present in a unit dose according to the invention. In an exemplary embodiment, 0.15 to 3.5% by weight of a resveratrol derivative are present in a unit dose according to the invention. In an exemplary embodiment, 0.2 to 2.5% by weight of a resveratrol derivative are present in a unit dose according to the invention. Doses of resveratrol employed for systemic treatment of adult humans will typically be in the range of about 100 mg to 1000 mg, preferably about 150 mg to 900 mg, preferably about 200 mg to 850 mg per day, in particular about 125 mg to 400 mg.

Studies demonstrate that resveratrol acts at many different levels including enhancing immunity, and decelerating ageing mechanisms. This results in its anti-obesity effects and specific interventions that hinder or mitigate conditions like diabetes, neurodegenerative disorders, and cardiovascular diseases. Significantly, thorough research consistently underscores its inhibitory role in combating cancer. Resveratrol serves as a chemopreventive agent across the four primary phases of carcinogenesis: initiation, promotion, progression, and metastasis. Resveratrol also influences thyroid function by enhancing iodide trapping (Duntas, L.H. J Endocrinol Invest. 2011). Resveratrol has been shown to inhibit the activation of NF-κB. This inhibition can occur through multiple mechanisms, including the prevention of IκB kinase (IKK) activation. IKK is responsible for the phosphorylation and subsequent degradation of IκB, an inhibitor of NF-κB. By preventing IKK activation, resveratrol can block the release and nuclear translocation of NF-κB, thereby reducing its activity (Ren et al. Pharmazie. 2013; Gonzalez et al. Nutr Metab. 2008). NF-κB has been implicated in the regulation of deiodinase 1 (D1) expression. TNF-α, a pro-inflammatory cytokine, activates NF-κB, which in turn inhibits T3-induced expression of D1. This suggests a role for NF-κB in modulating thyroid hormone metabolism, particularly in conditions like non-thyroidal illness syndrome (NTIS), where cytokines and oxidative stress are involved (Mancini et al. Mediators Inflammation. 2016).

*Monounsaturated fatty acids (MUFA)* - Monounsaturated fatty acids (MUFAs) are a class of fatty acids that are found in foods such as olive oil, nuts, and avocadoes. In particular this category includes: a) oleic acid, found mainly in olive oil but is also present in other vegetable oils, nuts, and seeds. b) palmitoleic acid: present in smaller amounts in some vegetable oils, fish, and meat. c) gadoleic acid: found primarily in sunflower and safflower seed oils. In the context of tumour progression, both pro- and anti-carcinogenic effects have been reported. MUFAs may also indirectly influence tumour progression by modulating tumour-promoting inflammation. Oleic acid is able to counteract the pro-inflammatory effects of saturated fatty acid, stearic acid, in human aortic endothelial cells in vitro and specifically in the context of cancer. Recent in vitro evidence shows that treatment of mouse hepatic cell lines with a combination of oleic acid and palmitic acid increases Dio1 mRNA expression (Bruinstroop et al. Molecular Metabolism. 2021).

This invention encompasses compositions which are formulated to contain elevated levels of the MUFAs selected from the group composed of oleic acid (C18:1n-9), gadoleic acid (20:1 n-11), palmitoleic (hexadecenoic) acid (C16:1n-7) and its positional isomers C16:1n-6, C16:1n-5, C16:1n-4, and C16:1n-3, myristoleic (tetradecenoic) acid (C14:1n-5) and its positional isomers C14:1n-4 and C14:1n-3, and lauroleic (dodecenoic) acid (C12:1n-3), or their mixtures, whether as the free acids, salts, or esters thereof. Hereinafter, the above MUFAs are sometimes referred to as "short chain MUFAs" and the levels of short chain MUFAs comprised in the composition are sufficiently high to produce beneficial improvements in the metabolic processing of lipids or glucose in animals to which these compositions of matter are regularly administered. The compositions of the present invention are formulated in the sense that the fatty acid content is adjusted to provide a sufficient amount of the short chain MUFAs. A "sufficient amount" of the short chain MUFAs in any given composition is determined in relation to the total amount of the said short chain MUFAs required for regular administration in order to produce the particularly desired beneficial improvement. In exemplary embodiments, the MUFA is oleic acid and its content is of about 0.1 g to 20 g, of about 0.5 g to 10 g, of about 3 g to 7 g, of about 5 g, of about 2.5 g, of about 1.7 g, of about 0.85 g.

*Taurine* - also known as 2-aminoethanesulfonic acid, is an amino acid extensively present in the tissues of all mammals. Numerous investigations have showcased the anti-inflammatory, antioxidant, and hypoglycemic attributes of taurine. In recent findings, taurine has been observed not only to alleviate the adverse repercussions of chemotherapy in cancer treatment, but also to exhibit antitumor characteristics. This includes the hindrance of cancer cell proliferation and the initiation of apoptosis in specific cancer types through the distinct regulation of proapoptotic and antiapoptotic proteins. Recent evidence shows that taurine supplementation inhibits muscle atrophy and could act against muscle loss in sarcopenia patients. (Doss HM, et al., Adv Exp Med Biol. 2022)

As used herein, "taurine" also encompasses taurine precursors, metabolites, derivatives and analogues of taurine which may be collectively termed "taurine-like substances". As used herein "taurine precursors" encompass substances that, when they are administered to a human or an animal, can be transformed, directly or indirectly, into taurine. As used herein, "taurine metabolites" encompass substances that are produced in vivo by trans formation of taurine. As used herein, "taurine derivatives" encompass substances that are structurally close to taurine but possess at least one structural difference, such as one or more chemical changes, e.g. at least one replacement of an atom or a chemical group found in taurine by a distinct atom or a distinct chemical group. As used herein, "taurine analogues" encompass substances that are chemically distinct from taurine but which exert the same biological activity.

In a particular embodiment, taurine precursors are selected from the group consisting of cysteine, cystathionine, homocysteine, S-adenosylhomocysteine, serine, N-acetyl-cysteine, glutathione, N-formylmethionine, S-adenosylmethionine, betaine and methionine. In another particular embodiment, taurine metabolites are selected from the group consisting of hypotaurine, thiotaurine, taurocholate, tauret also known as retinyliden taurine ((3,7-dimethyl-9-(2,6,6-trimehyl-1-cyclohexen-1-yl)-2,4,6,8-nonatetraen-1 -imido-(N-ethane sulfonic acid)). In another particular embodiment, taurine derivatives are selected from different entities including the group consisting of acetylhomotaurinate, and piperidino-, benzamido-, phthalimido- or phenylsuccinylimido taurine derivatives. Such taurine derivatives are described notably by Kontro et al. (1983) and by Andersen et al. (1984). Derivatives include for instance taurolidine (4 ,4'-methylene-bis(tetrahydro-2H-1,2,4-thiadiazine-1,1-dioxide or taurolin), taurultam and taurinamide, chlorohydrate-N-isopropylamide-2-(l-phenylethyl)aminoethanesulfonic acid. In another particular embodiment taurine analogs are selected from the group consisting of (+/-) piperidine-3 -sulfonic acid (PSA), 2-aminoethylphosphomc acid (AEP), (+/-)2-acetylaminocyclohexane sulfonic acid (ATAHS), 2-aminobenzenesulfonate (ANSA), hypotaurine, ± trans-2-aminocyclopentanesulfonic acid (TAPS) 8-tetrahydroquinoleine sulfonic acid (THOS), N-2-hydroxyethylpiperazine-N'-2-ethane sulphonic acid (HEPES), beta-alanine, glycine, guanidinoethylsulfate (GES), 3-acetamido-l-propanesulfonic acid (acamprosate).

In some embodiments, the dose of taurine is about 0.01 g to 3 g per day. In some embodiments, the dose of taurine is from about 0.02 to about 2 g per day. In some embodiments, the dose of taurine is equal to about 0.08, 0.1, 0.15, 0.16, 0.20, 0.25, 0.4, 0.5, 0.6, 1.5, 2, 2.5, 3, 3.5, 4, 4.5, 5, 5.5 or 6 g per day or within a range defined by any two of the aforementioned values.

*Selenocysteine* - Selenoproteins mainly have oxidoreductase functions but are also involved in many different molecular signalling pathways, physiological roles, and complex pathogenic processes (including, for example, teratogenesis, neurodegenerative, immuno-inflammatory, and obesity development). Deiodinases are a family of selenoproteins. Selenium concentration regulates activity and expression of the p27 substrate-binding subunit of type I 5'deiodinase. Selenium deficiency prevents type I deiodinase expression and may thus impair thyroid hormone action (Oertel et al. Am J Clin Nutrition 1993). DIO1 contains the rare amino acid selenocysteine at its active site and selenocysteine is required for proper DIO1 functionality (Rodriguez-Ruiz et al. Int J Mol Sci. 2022). Selenocysteine is not one of the standard 20 amino acids and yet it is incorporated into DIO1 during translation of the mRNA by the ribosome.

The composition of the invention comprises selenium or a selenium-based compound, preferably being selected from a selenate salt and seleno-amino acid. The selenate salt is preferably selected from the group consisting of sodium selenate, potassium selenate, barium selenate and ammonium selenate, more preferably selected from sodium selenate and potassium selenate. The seleno-amino acid preferably includes selenomethionine or selenocysteine. Other organic selenium compounds include Se-adenosyl-selenohomocysteine, methylselenocysteine, selenocystathionine, and y-glutamyl-Se-methylselenocysteine. According to some embodiments, the composition of the invention contains selenium or selenium-based compound such as selenocysteine or selenomethionine, in an amount ranging from 0.0001 to 1% by weight, from 0.001 to 0.1% by weight, from 0.005 0.05%, for example 0.01% by weight. In some embodiments, the dose of selenocysteine is equal to about 0.01, 0.02, 0.03, 0.06, 0.10, 0.15, 0.2, 0.25, 0.3, 0.5 mg per day or within a range defined by any two of the aforementioned values. For example, the composition or supplement described herein may contain from 100 to 250 mcg, from 30 to 60 mcg of selenocysteine per dosage unit.

Further to the above core ingredients, the composition of the invention may include further ingredients including for example oligoelements and vitamins. In a preferred embodiment of the invention, the composition further comprises at least one, at least two, at least three of ingredients selected from vitamin D, iron (Fe), iodine (I) and zinc (Zn). Even more preferably the composition further comprises vitamin D, iron (Fe), iodine (I) and zinc (Zn).

The composition of the invention may also include, further to the core ingredients and, optionally, the further ingredient indicated above also one or more of the following ingredients: copper (Cu), manganese (Mn), fluorine (F), molybdenum (Mo), chromium (Cr), vitamin E, vitamin K, thiamine (vitamin B1), riboflavine (vitamin B2), niacine (vitamin B3), panthotenic acid, vitamin B6, folic acid, vitamin B12, biotin, vitamin C, choline. It is contemplated that the invention described herein will be used as a supplement to elderly subjects at high risk of malnutrition and with low levels of fiT3/fiT4 ratio or to adult patients affected by cancer and with low levels of fT3/fT4 ratio.

Exemplary cases of elderly subjects at high risk of malnutrition include, but are not limited to: subjects characterised by a combination of factors such as poor dietary intake, acute and chronic health conditions, and/or social isolation. They may or may not experience various degrees of weight loss, muscle weakness, reduced ability to perform daily activities, and signs of cognitive decline.

Exemplary cancers include, but are not limited to:
Breast:
   - Breast Cancer
Digestive System:
   - Colorectal Cancer
   - EsophagealCancer
   - Liver Cancer
   - Pancreatic Cancer
   - Stomach (Gastric) Cancer
Endocrine System:
   - Thyroid Cancer
   - AdrenalCancer
   - Pituitary Cancer
Female Reproductive System:
   - Ovarian Cancer
   - Cervical Cancer
   - Uterine (Endometrial) Cancer
Head and Neck:
   - Head and Neck Cancer
   - Oral Cancer
   - Throat Cancer
Lung and Respiratory System:
   - Lung Cancer
   - Mesothelioma
Male Reproductive System:
   - Prostate Cancer
   - Testicular Cancer
Musculoskeletal System:
   - Bone Cancer
   - Soft Tissue Sarcoma
Skin:
   - Melanoma
   - Non-Melanoma Skin Cancer (e.g., Basal Cell Carcinoma, Squamous Cell Carcinoma) Urinary System:
   - Bladder Cancer
   - Kidney Cancer
Central Nervous System:
   - Brain Tumors
   - Spinal Cord Tumors
Eye:
   - Eye Cancer (Retinoblastoma)
Immune System:
   - Hodgkin Lymphoma
   - Non-Hodgkin Lymphoma
   - Hematologic malignancies and lymphoproliferative disorders

The composition of the invention can be administered in combination with a nutritional a nutritional backbone formulated as a complete enteral food comprising a balanced and easy to digest fat blend, carbohydrate blend and protein blend. In the medical field, these nutritional complete enteral formulae are used to treat and manage patients with a variety of ailments that have difficulty digesting foods and have decreased nutrient intake.

An exemplary embodiment of the above nutritional enteral formula is reported in the table below:

| **Nutritional Backbone** | **Volume 100 mL** |
|---|---|
| Proteins | about 10 g |
| Fatty Acids | about 5 g |

| | |
|---|---|
| *Medium-chain triglycerides* | *about 2 g* |
| *Polyunsaturated fatty acids* | *about 2 g* |
| *Omega-3 fatty acids* | *about 500 mg* |
| *Eicosapentaenoic acid and docosahexaenoic acid* | *about 500 mg* |
| Carbohydrates | about 20 g |
| *Maltodextrins* | *about 8 g* |
| *Sucrose* | *about 12 g* |

The composition of the invention can include the above nutritional backbone or can be administered in combination with the above nutritional backbone.

The following provides definitions of various terms and expressions used in the present application to describe the invention. Some terms (e.g. vitamin A, resveratrol, composition, etc.) are recited identically in the description of different aspects or embodiments of the invention. It is to be understood that definitions of terms and expressions used in more than one aspect of the invention apply equally to any aspect of the invention described herein in which those terms and expressions appear. This applies equally regardless of where, i.e. which section, within the present application such terms are defined or discussed.

In this application, the use of the singular (e.g. "a" or "the") may include the plural unless specifically stated otherwise. Further, the use of the term "including" as well as other grammatical forms such as "includes" and "included", is not limiting.

As used herein the term "comprising" has the broad standard meaning "including", "encompassing", or "containing". It includes the explicitly recited element(s), and also allows, but does not require, the presence of other or any other element(s) not recited. In addition to this broad meaning, as used herein, the term "comprising" also encompasses the limiting meaning "consisting of", according to which only the explicitly recited element(s), and no other(s) are present. In addition, the term "comprising" also includes the meaning of "consisting essentially of", which means that other element(s) may be present beyond those explicitly recited, provided the additionally present element(s) does not alter the technical effect achieved by the explicitly recited element(s).

As used herein the term "about" when referring to a particular value, e.g. an endpoint or endpoints of a range, encompasses and discloses, in addition to the specifically recited value itself, a certain variation around the specifically recited value. Such a variation may for example arise from normal measurement variability, e.g. in the weighing or apportioning of various substances by methods known to the skilled person. The term "about" shall be understood as encompassing and disclosing a range of variability above and below an indicated specific value, said percentage values being relative to the specific recited value itself, as follows. The term "about" may encompass and disclose variability of ± 5.0%. The term "about" may encompass and disclose variability of ± 4.9%. The term "about" may encompass and disclose variability of ± 4.8%. The term "about" may encompass and disclose variability of ± 4.7%. The term "about" may encompass and disclose variability of ± 4.6%. The term "about" may encompass and disclose variability of ± 4.5%. The term "about" may encompass and disclose variability of ± 4.4%. The term "about" may encompass and disclose variability of ± 4.3%. The term "about" may encompass and disclose variability of ± 4.2%. The term "about" may encompass and disclose variability of ± 4.1%. The term "about" may encompass and disclose variability of ± 4.0%. The term "about" may encompass and disclose variability of ± 3.9%. The term "about" may encompass and disclose variability of ± 3.8%. The term "about" may encompass and disclose variability of ± 3.7%. The term "about" may encompass and disclose variability of ± 3.6%. The term "about" may encompass and disclose variability of ± 3.5%. The term "about" may encompass and disclose variability of ± 3.4%. The term "about" may encompass and disclose variability of ± 3.3%. The term "about" may encompass and disclose variability of ± 3.2%. The term "about" may encompass and disclose variability of ± 3.1%. The term "about" may encompass and disclose variability of ± 3.0%. The term "about" may encompass and disclose variability of ± 2.9%. The term "about" may encompass and disclose variability of ± 2.8%. The term "about" may encompass and disclose variability of ± 2.7%. The term "about" may encompass and disclose variability of ± 2.6%. The term "about" may encompass and disclose variability of ± 2.5%. The term "about" may encompass and disclose variability of ± 2.4%. The term "about" may encompass and disclose variability of ± 2.3%. The term "about" may encompass and disclose variability of ± 2.2%. The term "about" may encompass and disclose variability of ± 2.1%. The term "about" may encompass and disclose variability of ± 2.0%. The term "about" may encompass and disclose variability of ± 1.9%. The term "about" may encompass and disclose variability of ± 1.8%. The term "about" may encompass and disclose variability of ± 1.7%. The term "about" may encompass and disclose variability of ± 1.6%. The term "about" may encompass and disclose variability of ± 1.5%. The term "about" may encompass and disclose variability of ± 1.4%. The term "about" may encompass and disclose variability of ± 1.3%. The term "about" may encompass and disclose variability of ± 1.2%. The term "about" may encompass and disclose variability of ± 1.1%. The term "about" may encompass and disclose variability of ± 1.0%. The term "about" may encompass and disclose variability of ± 0.9%. The term "about" may encompass and disclose variability of ± 0.8%. The term "about" may encompass and disclose variability of ± 0.7%. The term "about" may encompass and disclose variability of ± 0.6%. The term "about" may encompass and disclose variability of ± 0.5%. The term "about" may encompass and disclose variability of ± 0.4%. The term "about" may encompass and disclose variability of ± 0.3%. The term "about" may encompass and disclose variability of ± 0.2%. The term "about" may encompass and disclose variability of 0.1%. The term "about", in reference to the particular recited value, may encompass and disclose that exact particular value itself, irrespective of any explicit mention that this exact particular value is included; even in the absence of an explicit indication that the term "about" includes the particular exact recited value, this exact particular value is still included in the range of variation created by the term "about", and is therefore disclosed. Unless stated otherwise, if the term "about" is recited before the first endpoint of a numerical range, this term refers to both the first endpoint of the range and the second endpoint of the range. For instance, a recited range of "about X to Y" should be read as "about X to about Y". The same applies for a recited range of weight ratios. For instance, a recited range of weight ratios of "about X:Y-A:B" should be read as a weight ratio of "(about X):(about Y) - (about A):(about B)".

Unless stated otherwise, the designation of a range in the present application using a hyphen ("-") separating two bracketing values X and Y, or two bracketing ratios, is to be understood as meaning and disclosing the specified range in which both endpoint values X and Y are included. The same applies to a range expressed as "from X to Y". Accordingly, the expressions of ranges as "X - Y", "of X to Y", "from X to Y", "of X - Y" and "from X - Y" are to be understood equivalently as meaning and disclosing a range encompassing the end value X, all values between X and Y, as well as the end value Y. In contrast, the designation of a range in the present application using the word "between" preceding two bracketing values X and Y, or two bracketing ratios, is to be understood as meaning and disclosing the specified range in which both endpoint values X and Y are excluded, but all values between the specified endpoint values X and Y are included.

As used herein, the term "composition" encompasses and discloses any physical entity comprising or consisting of or consisting essentially of the respective recited substances. The physical form of the composition is not restricted. For example, the term "composition" encompasses and discloses a powder in which the recited substances are each present in powder form. As a further example, the term "composition" also encompasses and discloses a liquid solution in which the recited substances are present in solubilized form. As a further example, the term "composition" also encompasses and discloses an emulsion in which the recited substances are present. As a further example, the term "composition" also encompasses and discloses a suspension in which the recited substances are present. In particular, the term "composition" may be a "pharmaceutical composition" as defined herein below, and may be formulated for a desired route of administration. The term "composition" also specifically refers to nutritional composition or food supplement. As used and disclosed herein, the term "composition" also may be a composition suitable for oral delivery, e.g. in the form of a tablet, including but not limited to an effervescent tablet or a multilayer tablet, a powder, e.g. in the form of a sachet, a hard capsule, a soft gel capsule, a syrup, a cachet, a troche, or a lozenge, a pastille, e.g. a gummy pastille, a salve, or a liquid preparation. The term "composition" may also be a composition suitable for delivery by a non-oral route, for example in the form of a suppository, a tablet, a hard capsule, a soft gel capsule, a cream, a gel, a patch or a liquid. Further dosage forms of the composition as well as referred routes of administration are set out herein below.

In the present context, the term "combination" refers to the coadministration of the claimed ingredients or to the accompaniment of the composition of the invention with a further therapeutic regime or nutritional supplement to be co-administered in a subject as part of a given treatment regime.

The invention will be illustrated by reference to the specific non limitative examples below, which are representative of the invention. It must be understood, however, that the specific examples are provided only for the purpose of illustration, and that the invention may be practised otherwise than as specifically illustrated without departing from its spirit and scope.

### EXAMPLES

The present invention is directed to an improved nutritional supplement formulation, comprising vitamin A, resveratrol, oleic acid, taurine, selenocysteine. Those components can be grouped in a single product or associated to an integrated composition where a specific amount of proteins, carbohydrates and fats is included. Table 1 below provides a list and exemplary daily dosages of the core components of the invention.

**Table 1.**

| ***Core Invention Component*** | ***Daily Dosage*** | ***Range*** |
|---|---|---|
| Vitamin A | 750 mcg | 500-1000 mcg |
| Resveratrol | 400 mg | 300-500 mg |
| Oleic Acid | 5g | 3-7 g |
| Taurine | 500 mg | 100-1000 mg |
| Selenocysteine | 200 mcg | 100-250 mcg |

The compositions can be administered in various forms, such as tablets, capsules, granules, powders, solutions, suspensions, emulsions, ointments. The dosage forms of the compositions can be tailored to the desired mode of administration of the compositions. For oral administration, the compositions can take the form of, e.g., a powder, tablet or capsule (including soft-gel capsule), or can be, e.g., an aqueous or nonaqueous solution, suspension or syrup. Tablets and capsules for oral administration can include one or more commonly used excipients, diluents and carriers, such as mannitol, lactose, glucose, sucrose, starch, corn starch, sodium saccharin, talc, cellulose, magnesium carbonate, and lubricating agents (e.g., magnesium stearate, sodium stearyl fumarate). If desired, flavouring, colouring and/or sweetening agents can be added to the solid and liquid formulations. Other optional ingredients for oral formulations include without limitation preservatives, suspending agents, and thickening agents.

### Example 1 - Soft-gel capsules. 1000 mg or 2000 mg final products.

1000 mg capsules containing: Vitamin A 0,125 mg, Resveratrol 67 mg, Oleic Acid 850 mg, Taurine 83 mg, Selenocysteine 0,03 mg. Indicative posology 2 capsules/tid at regular intervals before meals.

2000 mg capsules containing: Vitamin A 0,250 mg, Resveratrol 134 mg, Oleic Acid 1700 mg, Taurine 166 mg, Selenocysteine 0,06 mg. Indicative posology 1 capsule/tid at regular intervals before meals.

*Example 2* - Soluble Powder. 25 g or 50 g final products. To be diluted in 150 or 250 ml of water. Complete nutritional formulas including a variable amount of proteins, carbohydrates and fats + core invention product components at the following dosages for an exemplifying tid administration of Vitamin A 0,250 mg, Resveratrol 134 mg, Oleic Acid 1700 mg, Taurine 166 mg, Selenocysteine 0,06 mg.

*Example 3* - Drinkable Shake. 200 ml or 300 ml final products. Complete nutritional formulas including a variable amount of proteins, carbohydrates and fats + core invention product components at the following dosages for an exemplifying bid administration of Vitamin A 0,375 mg, Resveratrol 200 mg, Oleic Acid 2500 mg, Taurine 250 mg, Selenocysteine 0,100 mg.

Example 4 - Table 2 below summarises one exemplary composition of the nutritional supplement of the invention.

**Table 2.**

| **Component** | | **Dose for 100 ml product** |
|---|---|---|
| **Nutritional Backbone** | | |
| Proteins | | 10 g |
| Fatty Acids | | 5 g |
| | *Medium-chain triglycerides* | *2 g* |
| | *Polyunsaturated fatty acids* | *2 g* |
| | *Omega-3 fatty acids* | *500 mg* |
| | *Eicosapentaenoic acid and docosahexaenoic acid* | *500 mg* |
| Carbohydrates | | 20 g |
| | *Maltodextrins* | *8 g* |
| | *Sucrose* | *12 g* |

| **Core** | | |
|---|---|---|
| Selenocysteine | | 200 µg |
| Retinol | | 750 µg-RAE |
| Taurine | | 500 mg |
| Resveratrol | | 400 mg |
| Oleic Acids | | 5g |

| **Additional Elements** | | |
|---|---|---|
| Iron (Fe) | | 2,1 mg |
| Zinc (Zn) | | 8 mg |
| Copper (Cu) | | 350 µg |
| Manganese (Mn) | | 0,63 mg |
| Fluorin (F) | | 0,19 mg |
| Molibdenum (Mo) | | 20 µg |
| Chromium (Cr) | | 13 µg |
| Iodine (I) | | 100 µg |
| Vit. D | | 2,1 µg |
| Vit. E | | 3,7 mg-α-TE |
| Vit. K | | 16 µg |
| Tiamine (Vit. B1) | | 0,45 mg |
| Riboflavine (Vit. B2) | | 0,5 mg |
| Niacine (Vit. B3) | | 3,6 mg-NE |
| Pantotenic acid | | 1,6 mg |
| Vit. B6 | | 0,53 mg |
| Folic acid | | 80 µg |
| Vit. B12 | | 2 µg |
| Biotin | | 12 µg |
| Vit. C | | 250 mg |
| Choline | | 110 mg |

### DISCUSSION

Despite progressively increasing attention from the scientific community on the importance of nutritional sustenance in elderly subjects and patients with advanced cancer, major needs are still unmet. In particular, i) innovative, easy-to-test and reliable criteria to select patients in need for support and ii) conceptualization and realisation of a specific product for intervention. The impact of fT3/fT4 ratio on the prognosis of elderly subjects and advanced cancer patients and the importance of nutritional intervention to restore a more balanced peripheral thyroid hormones conversion are the basis for the present patent application.

New data on file produced by the inventors strongly correlated the survival of patients with metastatic colorectal, advanced cervical cancer, metastatic breast and advanced lung cancer treated with modern immunotherapies and levels of fT3/fT4 ratio. Those data were needed to validate and further confirm in as many different settings as possible the initial data published and publicly available, as well as for gathering new relevant information on patients' characteristics and prognosis.

As fT3/fT4 ratio is regulated by enzymes that modify the T4 form into T3, work has been done to compose a supplement that can sustain a progressive improvement in deiodinase activity. The objective is to provide patients with all the nutritional elements supporting metabolic induction, especially hepatic production of deiodinase 1, correcting the peripheral conversion defect of these hormones and restoring a more physiological condition capable of supporting the organism and improving the effectiveness of necessary therapies. In addition to the metabolic benefits, the induction of liver deiodinase activity by specific nutrients restores the fundamental general effects related to the endocrine system such as muscle trophism (anabolism), immune system activity, cognitive function etc.

The nutritional support provided by the invention could offer patients greater chances of survival, providing better response possibilities to treatments, especially immunotherapies, and simultaneously improving the quality of life for patients.

## Claims

1. A composition or combination comprising:
• vitamin A; and
• resveratrol or derivatives thereof; and
• a monounsaturated fatty acid or mixtures thereof; and
• taurine or taurine-like substances; and
• selenium or a selenium-based compound, preferably being selected from a selenate salt and seleno-amino acid.

2. The composition or combination according to claim 1 wherein the resveratrol is comprised in parts of a plant or in an extract of said parts of plant, wherein said plant is selected from Vitis vinifera, Polygonum cuspidatum, Blueberries (Vaccinium spp.), Mulberries (Morus spp.), Cranberries (Vaccinium macrocarpon), and Aronia (Photinia melanocarpa).

3. The composition or combination according to any of claims 1 and 2 wherein the monounsaturated fatty acid is selected from oleic acid, palmitoleic acid and gadoleic acid or mixtures thereof.

4. The composition or combination according to any of claims 1 to 3 wherein the seleno-aminoacid is selected from selenocysteine and selenomethionine, preferably the seleno-aminoacid is selenocysteine.

5. The composition or combination according to any one of previous claims comprising:
- about 125 to 1000 mcg of vitamin A; and/or
- about 50 to 500 mg of resveratrol; and/or
- about 1.5 to 7 g of oleic acid; and/or
- about 100 to 1000 mg of taurine; and/or
- about 30 to 250 mcg of selenocysteine.

6. The composition or combination according to any one of previous claims further comprising one or more ingredients selected from: vitamin D, iron (Fe), zinc (Zn) and iodine (I).

7. The composition or combination according to any one of previous claims further comprising one or more ingredients selected from: copper (Cu), manganese (Mn), fluorine (F), molybdenum (Mo), chromium (Cr), vitamin E, vitamin K, thiamine (vitamin B1), riboflavine (vitamin B2), niacine (vitamin B3), panthotenic acid, vitamin B6, folic acid, vitamin B12, biotin, vitamin C and choline.

8. The composition according to any one of previous claims further comprising or in combination with a nutritional supplement comprising proteins, carbohydrate and fatty acids, preferably said nutritional supplement comprises proteins in concentration of about 10g/100 mL, fatty acids in concentration of about 5g/100 mL and carbohydrates in concentration of about 20g/100 mL.

9. The composition according to any one of claims 1 to 8 further comprising at least one excipient or diluent, preferably for oral administration, preferably in the form of a lozenge, a hard capsule, a soft gel capsule, a powder, a syrup, a cachet, a pill, a tablet, a pastille, preferably said excipient and / or diluent, is selected from the group consisting of: calcium phosphate, dicalcium phosphate, microcrystalline cellulose, silicon dioxide, sucrose, gum arabic, corn starch, medium chain triglycerides, tricalcium phosphate, cross-linked sodium carboxymethylcellulose, hydroxypropylmethylcellulose, polyethylene glycol, titanium dioxide, polyvinylpyrrolidone, talc, erythritol, xylitol, steviol and sucralose glycosides, magnesium salt of fatty acids, preferably said magnesium salt of fatty acids is magnesium stearate.

10. Pharmaceutical composition, medical device or supplement or food product, preferably a food for special medical purposes, or product to drink, comprising the composition according to any one of claims 1 to 9.

11. The pharmaceutical composition, medical device or supplement or food product, preferably a food for special medical purposes, or product to drink according to claim 10 for use in the nutritional support or treatment of patients affected by cancer at high risk of malnutrition as defined by level of fT3/fT4 ratio below the mean reference values for said subjects.

12. The pharmaceutical composition, medical device or supplement or food product, preferably a food for special medical purposes, or product to drink according to claim 10 for use in the nutritional support and/or in the treatment and prevention of decreased skeletal mass of elderly subjects at high risk of malnutrition as defined by level of fT3/fT4 ratio below the mean reference values for said subjects.

13. The pharmaceutical composition, medical device or supplement or food product, preferably a food for special medical purposes, or product to drink for use according to any one of claims 11-12 **characterised in that** supports deiodinase 1 gene expression and enable deiodinase proteins synthesis and/or provides specific trace elements for deiodinase proteins synthesis and/or reduces the negative impact of inflammation on deiodinase expression and/or enhance immune system and improve muscle trophism and/or sustains fT4 to fT3 peripheral conversion.

14. The pharmaceutical composition, medical device or supplement or food product, preferably a food for special medical purposes, or product to drink according to claims 11 and 13 wherein said cancer includes breast cancer, colorectal cancer, esophageal cancer, liver cancer, pancreatic cancer, stomach (gastric) cancer, endocrine system cancer including thyroid cancer, adrenal cancer and pituitary cancer, female reproductive system cancer, including ovarian cancer, cervical cancer and uterine (endometrial) cancer, head and neck cancer, oral cancer, throat cancer, lung cancer, mesothelioma, male reproductive system cancer, including prostate cancer and testicular cancer, musculoskeletal system cancer, including bone cancer and soft tissue sarcoma, skin cancer, including melanoma and non-melanoma skin cancer such as basal cell carcinoma and squamous cell carcinoma, urinary system cancer including bladder cancer and kidney cancer, central nervous system cancer including brain tumors and spinal cord tumors, eye cancer (retinoblastoma), immune system cancer including Hodgkin lymphoma and non-Hodgkin lymphoma.

15. Non therapeutic use of the composition according to any one of claims 1-9 in the nutraceutical sector or as base ingredient in the preparation of supplements and/or devices and/or food products and/or foods for special medical purposes.

16. The composition according to any one of claims 1-9, or the pharmaceutical composition, medical device or supplement or food product, preferably a food for special medical purposes, or product to drink, according to claim 10 for use according to any one of claims 11-14 in combination with a further therapeutic agent or therapeutic intervention or with nutritional supplement.
